**Europäisches Patentamt**

**European Patent Office**

**Office européen des brevets**

(19)

(11) **EP 1 308 713 A1**

(12) **EUROPEAN PATENT APPLICATION**

(43) Date of publication:
**07.05.2003 Bulletin 2003/19**

(51) Int Cl.$^7$: **G01N 21/31**, G01N 21/35, G01N 33/14

(21) Application number: **01204187.7**

(22) Date of filing: **02.11.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Qmet ApS**
**2942 Skodsborg (DK)**

(72) Inventors:
• **Pedersen, Thorvald**
**2610 Rodovre (DK)**
• **Henningsen, Jes**
**3660 Stenlose (DK)**

(74) Representative: **Nielsen, Henrik Sten et al**
**Budde, Schou & Ostenfeld A/S**
**Vester Søgade 10**
**1601 Copenhagen V (DK)**

(54) **A method of determining the content of SO2 in a beverage product**

(57) A method of determining the content of $SO_2$ in a beverage product contained within a process line, such as beer, wine, champagne, fruit juice, etc., the method comprises: extracting a sample of a specific volumetric size of the beverage product from the process line, injecting the sample to a sealed sample container defining a specific inner volume exceeding the specific volumetric size of the sample for the generation of a gaseous headspace above the sample within the sealed sample container, allowing the sample to establish in the gaseous headspace a concentration of $SO_2$ representative of the content of $SO_2$ of the beverage product, transferring a fraction of the gaseous headspace to an IR measuring apparatus, measuring the content of $SO_2$ of the fraction of the gaseous headspace by means of the IR measuring apparatus, and determining the content of $SO_2$ of the beverage product on the basis of the measurement of the content of $SO_2$ of the fraction of the gaseous headspace, preferably the measurement of the content of $SO_2$ is performed as a determination of the attenuation of transmission through the fraction at one or more IR wavelengths.

EP 1 308 713 A1

**Description**

The present invention relates to a method of determining the content of SO$_2$ in a beverage product. In particular, the present invention relates to a novel technique of determining the content of SO$_2$ in beer or wine.

In beverage products, in particular beer, a small percentage of SO$_2$ is present in the product. For various reasons including the taste of the product and the obligation of informing of the content of the product, the content of SO$_2$ should be kept to less than 10 ppm. On the other hand, for obtaining the preservative effect of SO$_2$ in the beverage product, the content of SO$_2$ should be maintained as high as possible and therefore, in the production of in particular beer, it has turned out to be extremely important to be able to monitor the content of SO$_2$ for maintaining the content of SO$_2$ at exactly 10ppm.

In the prior art technique, various approaches have been used for the determination of SO$_2$ in beverage products and in this context, reference is made to DE 1970690, US 5,844,123, US 5,614,718, US 5,426,593 and US 4,315,754. The above US patents are further incorporated in the present specification by reference.

In the above DE reference, the content of SO$_2$ of a liquid is determined by use of a light source generating visible light, in particular visible light of a wavelength of 590 nm.

In US 5,844,123, the content of gas constituents is determined by means of a pH measuring system, in particular a pH sensor of the type pH sensitive FET.

In US 5,614,718, a non-invasive technique of determining at least one gaseous constituent in the headspace above a liquid contained within a container is described according to which technique, NIR spectral analysis is used. The technique is particularly developed for the determination of the content of SO$_2$.

In US 5,426,593, the content of a gas, in particular O$_2$ or alternatively CO$_2$ and N$_2$ of a container also including a liquid is described. The measuring technique is based on IR absorption measurement.

US 4,315,754 describes a fluid injection analysis technique for the determination of SO$_2$ in wine based on the well-known West-Gaecke method.

As distinct from the above prior art measuring techniques used for the determination of the content of gaseous constituents in liquids, in particular beverage products such as beer, wine etc., the present invention is based on the realisation that a highly accurate and reliable determination of the content of SO$_2$ in a beverage product, in particular in beer or wine may be established by the use of IR measuring techniques.

It is contemplated that the usage of IR measuring techniques for the determination of the content of SO$_2$ in a beverage product, in particular beer or wine due to its high discrimination between various components and constituents allow for a more accurate and reliable determination as compared to the prior art measuring techniques described in the references listed above.

It is further contemplated that the novel technique according to the present invention allows for a substantial real time and on-line monitoring of the content of SO$_2$ in a beverage product contained within a process line allowing the content to be continuously monitored and adjusted, if necessary, for complying with specific minimum or maximum content requirements such as the above described conflict between on the on hand the maintenance of a high content of SO$_2$ for obtaining the preservation advantage and on the other hand the reduction of the content of SO$_2$ for obtaining advantages as to taste and compliance with statutory requirements as to the obligation of informing on the product about the presence of constituents present in an amount above a specific lower limit such as a limit 10 ppm.

The above objects, features and advantages together with numerous other objects, advantages and features which will be evident from the below detailed description of the present invention is according to the teachings of the present invention obtained by a method of determining the content of SO$_2$ in a beverage product contained within a process line, such as beer, wine, champagne, fruit juice, etc., the method comprising:

a) extracting a sample of a specific volumetric size of the beverage product from the process line,
b) injecting the sample to a sealed sample container defining a specific inner volume exceeding the specific volumetric size of the sample for the generation of a gaseous headspace above the sample within the sealed sample container,
c) allowing the sample to establish in the gaseous headspace a concentration of SO$_2$ representative of the content of SO$_2$ of the beverage product,
d) transferring a fraction of the gaseous headspace to an IR measuring apparatus,
e) measuring the content of SO$_2$ of the fraction of the gaseous headspace by means of the IR measuring apparatus, and
f) determining the content of SO$_2$ of the beverage product on the basis of the measurement of the content of SO$_2$ of the fraction of the gaseous headspace.

Generally, the present invention is based on a measuring technique, by which SO$_2$ is measured through absorption of monochromatic radiation generated by non-linear interactions.

According to the basic teachings of the present invention, the content of $SO_2$ is determined in the beverage product by determining the content of $SO_2$ in a headspace above a sample which is received within a sealed sample container. By the provision of the sample within the sealed sample container, the sample may be processed for various purposes as will be described in greater details below or simply be allowed to rest for the generation of a state of equilibrium between the headspace and the sample in order to allow for an accurate determination of the content of $SO_2$ in the beverage product based on the determination or measurement of the content of $SO_2$ in the gaseous headspace containing $SO_2$.

According to alternative embodiments of the method according to the present invention, the extraction of the sample from the production line may be carried out by means of a sample collector device such as a syringe or any other sample extraction apparatus or alternatively be produced by means of a separate tube or pipeline connected to the process line and including controllable valves for the extraction of the sample of the specific volumetric size from the process line without the use of separate automated or manually operated devices or apparatuses.

Provided a separate extraction device or apparatus be used, the injection of the sample into the sealed sample container may be performed manually or automated through appropriate closure valves or similar devices for preventing the introduction of constituents into the sealed sample container which constituents might influence the determination or measurement of the $SO_2$ content. Provided a pipeline or tube connected to the process line be used, the injection of the sample into the sealed sample container is performed by simply controlling input valves communicating with the pipeline for allowing the sample to be introduced into the sample container.

The step of allowing the sample to establish in the gaseous headspace a concentration of $SO_2$ representative of the content of $SO_2$ in the beverage product may, dependent on the circumstances as will be described in greater details below, be performed in numerous ways further dependent on the actual content of the beverage product in question.

The transfer of a fraction of the gaseous headspace to the IR measuring apparatus may, like the extraction of the sample from the process line, be performed in an on-line system or alternatively by means of a separate transferring device such as a syringe or the like. Further, as far as the IR measuring technique itself is concerned, various transmission, absorption or refractive measuring techniques may be used as will also be described in greater details.

The determination of the content of $SO_2$ of the beverage product on the basis of the measurement of the content of $SO_2$ of the fraction of the gaseous headspace may be performed based on a reference sample analysis or alternatively based on a calculation routine based on basic physical conditions and realisations.

According to a first embodiment of the method according to the present invention, the measurement of the content of $SO_2$ is performed as a determination of the attenuation of a monochromatic beam of infrared light in a specific wavelength range such as the 7.4 µm wavelength range which light beam is generated as the difference frequency between radiation from two diode lasers oscillating around two different NIR frequencies, preferably within the range 1200-1400 nm and 1400-1700 nm, respectively, such as 1290 nm and 1562 nm, respectively. As mentioned above, the IR measuring technique may be based on the determination of transmission or alternatively the determination of absorption or attenuation of transmission or any other transmission, absorption or refractive measuring technique.

For providing a reliable measurement of the content of $SO_2$ of the fraction of the gaseous headspace by means of the IR measuring apparatus, the determination of the attenuation of transmissions through the fraction preferably further involves the comparison of attenuation of transmission of IR through the fraction relative to the attenuation of transmission through atmospheric air or alternatively vacuum.

According to two distinctly different measuring techniques, the measurement of the content of $SO_2$ of the fraction of the gaseous headspace may be performed by means of a laser spectroscope or alternatively by means of an infrared monitor. Infrared monitors are commercially available from several manufacturers. At present it is contemplated that the use of the laser spectroscope is preferable for the reason that no elaborated preparation of the sample be needed except for the adjustment of the pH of the sample to 1.0 for converting all S(IV) to $SO_2$.

Provided the infrared monitor be used, the step of allowing the sample to establish in the gaseous headspace a concentration of $SO_2$ representative of the content of $SO_2$ of the beverage product necessitates further the removal of constituents such as $CO_2$ from the sample which constituents might else influence the IR measurement of step e). For removing $CO_2$ from the sample, the pH of the sample is initially adjusted to a fairly low value such as a value of 4-6, e.g. 5-6, preferably approximately 5.5 whereupon the headspace is preferably flushed with $N_2$ for the removal of gaseous $CO_2$ from the headspace. Provided the beverage products include aromatic products or constituents, these constituents may also be removed like $CO_2$ provided the infrared monitor be used and for the removal of the aroma products or constituents from the beverage product, the method according to the present invention further includes the step of adjusting the pH of the sample after the removal of $CO_2$ to about 7.0 and optionally the step of causing the remaining constituents such as the aroma products to evaporate in vacuum.

The step of allowing the sample to rest is preferably performed for a specific predetermined period of time, such as a period of time of 5-15 min.

The use of two or more IR wavelengths is preferably performed by the use of a measuring apparatus having two or more distinct IR wavelengths, such as a wavelength of approximately 1200-1400 nm, e.g. 1250-1350 nm, preferably approximately 1290 nm and a wavelength of approximately 1400-1700 nm, e.g. 1500-1600 nm, preferably approximately 1562 nm.

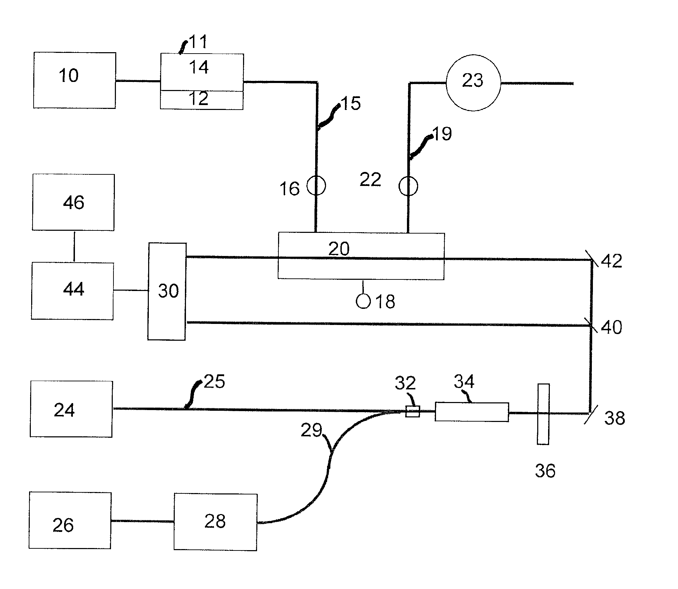

The invention is now to be further described with reference to the drawing in which a schematic prototype implementation of a system for carrying out the method according to the present invention is illustrated.

In the drawing, a schematic system for carrying the method according to the present invention is shown. The method of determining or measuring the content of $SO_2$ in a beverage product is based on the detection of attenuation of a monochromatic beam of infrared light in the 7.4 $\mu$m wavelength range as the monochromatic beam is transmitted through the $SO_2$ containing gas mixture along a known wavepath having a specific length.

The light beam is generated with the difference frequency from two diode lasers oscillating at 1290 nm and 1562 nm, respectively. The system includes in greater details a liquid reservoir 10 from which a liquid sample is transferred to a sealed chamber 11 in which the liquid which is preferably pre-treated before the IR measurement, is contained. The liquid contained within the seal chamber 11 is designated the reference numeral 12. Above the liquid 12, a headspace is provided in which headspace a gas is present, which gas includes $SO_2$ originating from the pre-treated liquid 12 and further from the liquid contained within the liquid reservoir 10.

From the sealed chamber 11, a fraction of the gas of the headspace 14 is sucked through a pipe 15 in which a controllable closure valve 16 is present. The pipe 16 transfers the fraction of the gas of the headspace 14 to a multipass cell 20 in which the fraction is exposed to IR radiation as will be described in greater details below.

From the multipass cell 20, a further pipe 21 provides air communication to a diaphragm pump 23 through a controllable closure valve 22 similar to the controllable closure valve 16 described above. The multipass cell 20 is further provided with a pressure gauge 18 and constitutes the central measuring chamber in which the attenuation of transmission of IR due to the presence of $SO_2$ is measured.

The above described components basically constitute the components of the system serving the purpose of the extraction of a liquid sample and of preparing the liquid sample for the IR measurement in the multipass cell. The transfer of the gas from the headspace 14 of the sealed chamber 11 is as will be readily understood by a person having ordinary skill in the art controlled by closing and opening the valves 16 and 22 and operating the diaphragm pump 23 while at the same time monitoring the pressure within the multipass cell 20 by means of the pressure gauge 18.

In the lower part of the drawing, two lasers 24 and 26 are shown, generating 1290 nm IR radiation and 1562 nm IR radiation, respectively. The output of the laser 26 generating the 1562 nm IR radiation is connected to a C-band fiber amplifier 28 further connected to an optical fiber 29. Similarly, the output of the laser 24 generating the 1290 nm IR radiation is connected to a further optical fiber 25. The optical fibers 25 and 29 are jointly connected to a graded index lens 32 which further optically communicates with a non-linear crystal 34.

From the non-linear crystal 34, radiation at the difference frequency of radiation originating from the lasers 24 and 26 of a wavelength of 1290 nm and 1562 nm, respectively, is input to a filter 36 and radiated towards a mirror 38 from which the IR radiation is directed to a beam splitter 40 serving the purpose of splitting the IR radiation into two parts, the first part being directed to a further mirror 42 directing the IR radiation through the multipass cell 20 and a second part being directed past the multipass cell and constituting a reference IR radiation. The reference IR radiation and the radiation transmitted through the multipass cell 20 and output therefrom are input to a balanced detector 30.

The electrical output of the balanced detector 30 is connected to a measuring apparatus 44 which is further connected to a display 46. The measuring apparatus 44 and the display 46 are preferably constituted by a PC based measuring set-up in which the measuring signals from the balance detector Q are input to an AD converter and processed within the PC for the generation of a figure representing the content of $SO_2$ of the liquid contained within the liquid reservoir 10. The figure may, based on the software of the PC, be presented in any relevant form as a graphical representation illustrating any variation in the $SO_2$ content as compared to a previous measuring routine and the conversion of the measuring results or signals output from the balance detector 30 into the figure representing the content of $SO_2$ of the liquid contained within the liquid reservoir 10 is performed based on a calculation and conversion program in which empirical data and optionally also physical parameters are used for the conversion.

The physical background of the measurement principle is presented in Appendix A.

Reference is further made to:

[1] B. Sumpf, D. Rehle, T. Kelz, H.D. Kronfeldt, "A tunable diode-laser spectrometer for the MIR region near 7.2 $\mu$m applying difference-frequency generation in $AgGaSe_2$", Applied Physics B 67. 369-373 (1988)

**Certain advantages of the measuring system**

The necessary discrimination against interfering molecules which are present in the gas mixture at much higher concentrations is obtained by regulating the valves 16 and 22 to a suitable flow rate at a pressure in the 10-100 mbar range in the cell. If desirable, the measurements can be performed with closed cell.

Data processing is performed with standard AD/DA data acquisition and subsequent numerical analysis with a PC. The result of the analysis is presented at the screen of the PC as a value for the concentration including the associated uncertainty, and an optional absorption spectrum which will allow the operator to asses operation of the system.

Depending on the stability of the transmitted signal, the detection may involve wavelength modulation of one of the lasers and lock-in detection of the detected signal at a harmonic of the modulation frequency. This is a standard technique.

the system in principle does not require calibration since the measurement result is based on a relative measurement (the ratio of the light intensity before and after the cell) and a set of known constants. However, the system can optionally be provided with a calibration cell with a known $SO_2$ concentration for verification of the sensitivity.

**Appendix A**

Physical background of the measurement principle.

The wavelength of the light is tuned to the vicinity of an absorption of $SO_2$ in the wavelength range around 7.4 µm. The attenuation of the light beam is given by beer's law:

$$I(x) = I(0)\exp(-\alpha x)$$

where I is the intensity of the light, s is the distance travelled, and $\alpha$ is the absorption coefficient, given by the expression:

$$\alpha = NSg(v-v_0)$$

S is a molecular parameter denoted the line strength, and is characterised of the chosen line, and $g(v-v_0)$ is a mathematical function which specifies the variation of the absorption coefficient over frequencies v in the vicinity of the line center frequency $v_0$. S and $g(v-v_0)$ are known from the literature.

The remaining factor N is the volume density of the target molecule $SO_2$, which at a given total pressure p is related to the concentration c through the expression:

$$N = cp/kT$$

where k is the Boltzmann constant and T is the absolute temperature.

Thus, knowing S, $g(v-v_0)$, L, p and T, a measurement of the light attenuation will yield the concentration c.

**List of references**

Liquid reservoir        10
Sealed chamber          11
Pre-treated liquid      12
Headspace               14
Pipe                    15
Valve                   16
Pressure gauge          18
Pipe                    19
Multipass cell          20
Pipe                    21
Valve                   22
Diaphragm pump          23
1290 nm laser           24
Optical fiber           25
1562 nm laser           26
C-band fiber amplifier  28
Optical fiber           29

| Balance detector | 30 |
| Graded index lens | 32 |
| Non-linear crystal | 34 |
| Filter | 36 |
| Mirror | 38 |
| Beam splitter | 40 |
| Mirror | 42 |
| Measuring apparatus | 44 |
| Display | 46 |

Although the present invention has been described above with reference to a specific system for carrying out the method according to the present invention, the invention is by no means to be construed as limited to the above-described system but rather to be interpreted in the broad scope and sense of the appending claims. Further numerous modifications and alterations are obvious to a person having ordinary skill in the art and such modifications and alterations are consequently to be considered part of the present invention.

**Claims**

1.   A method of determining the content of $SO_2$ in a beverage product contained within a process line, such as beer, wine, champagne, fruit juice, etc., said method comprising:

   a) extracting a sample of a specific volumetric size of said beverage product from said process line,
   b) injecting said sample to a sealed sample container defining a specific inner volume exceeding said specific volumetric size of said sample for the generation of a gaseous headspace above said sample within said sealed sample container,
   c) allowing said sample to establish in said gaseous headspace a concentration of $SO_2$ representative of the content of $SO_2$ of said beverage product,
   d) transferring a fraction of said gaseous headspace to an IR measuring apparatus,
   e) measuring the content of $SO_2$ of said fraction of said gaseous headspace by means of said IR measuring apparatus, and
   f) determining the content of $SO_2$ of said beverage product on the basis of said measurement of the content of $SO_2$ of said fraction of said gaseous headspace.

2.   The method according to claim 1, said measurement of the content of $SO_2$ being performed as a determination of the attenuation of transmission through said fraction at one or more IR wavelengths.

3.   The method according to claim 2, said measurement of the content of $SO_2$ of said fraction of said gaseous headspace my means of said IR measuring apparatus involving the comparison of attenuation of transmission of IR through said fraction relative to the attenuation of transmission through atmospheric air or alternatively vacuum.

4.   The method according to any of the claims 1-3, said measurement of the content of $SO_2$ of said fraction of said gaseous headspace being performed by means of a laser spectroscope, said measuring apparatus at two or more distinct IR wavelengths, such as a wavelength of 1290 nm and a wavelength of 1562 nm.

5.   The method according to any of the claims 1-3, said measurement of the content of $SO_2$ of said fraction of said gaseous headspace being performed by means of an infrared monitor.

6.   The method according to any of the claims 1-5, said measurement of the content of $SO_2$ of said fraction of said gaseous headspace being performed at two or more distinct IR wavelengths such as a wavelength of approximately 1200-1400 nm, e.g. 1250-1350 nm, preferably approximately 1290 nm and a wavelength of approximately 1400-1700 nm, e.g. 1500-1600 nm, preferably approximately 1562 nm.

7.   The method according to any of the claims 1-6, said step c) being performed by allowing said sample to rest for a specific predetermined period of time, such as a period of time of 5-15 min.

8.   The method according to any of the claims 1-7, further including the additional step included in step c) of removing constituents such as $CO_2$ from said sample which might influence the IR measurement of step e).

9.  The method according to claim 8, said step of removing said constituent, such as $CO_2$ including adjusting the pH of the sample to a specific low value, such as a value of 4-6, e.g. 5-6, preferably approximately 5.5 for the removal of $CO_2$ from the sample and optionally flushing said headspace with $N_2$ for the removal of gaseous $CO_2$ from headspace.

10. The method according to claim 9, further including the step of adjusting the pH of said sample after said removal of $CO_2$ to about 7.0, and optionally the step of causing the remaining constituents to evaporate in vacuum.

11. The method according to any of the claims 1-10 including the step of adjusting the pH of said fraction prior to the measurement of the content of $CO_2$ of said fraction to a low value, such as a value of 1.0.

**EP 1 308 713 A1**

**European Patent Office**

# EUROPEAN SEARCH REPORT

**Application Number**

EP 01 20 4187

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | US 5 844 123 A (SMITH RODNEY EDWARD ET AL) 1 December 1998 (1998-12-01) * column 5, line 33 - line 11 * * column 11, line 66 - column 12, line 12 * --- | 1 | G01N21/31 G01N21/35 G01N33/14 |
| A | WO 93 17324 A (PROCAL ANALYTICS) 2 September 1993 (1993-09-02) * abstract * * page 3, paragraph 4 - page 5, paragraph 4 * * figure 1 * --- | 1 | |
| A | US 3 942 356 A (BRANSCOMBE RICHARD ALAN ET AL) 9 March 1976 (1976-03-09) * column 4, line 30 - line 39 * --- | 1 | |
| A | US 6 138 497 A (KUGLER RICHARD E ET AL) 31 October 2000 (2000-10-31) * column 3, line 53 - column 4, line 12 * --- | 1 | |
| D,A | SUMPF B ET AL: "A TUNABLE DIODE-LASER SPECTROMETER FOR THE MIR REGION NEAR 7.2 MUM APPLYING DIFFERENCE-FREQUENCY GENERATION IN AGGASE2" APPLIED PHYSICS B: LASERS AND OPTICS, SPRINGER INTERNATIONAL, BERLIN, DE, vol. B67, no. 3, 1 September 1998 (1998-09-01), pages 369-373, XP000783017 ISSN: 0946-2171 * abstract * ----- | 2-6 | **TECHNICAL FIELDS SEARCHED** (Int.Cl.7) G01N |

The present search report has been drawn up for all claims

| Place of search | Date of completion of the search | Examiner |
|---|---|---|
| THE HAGUE | 8 May 2002 | Verdoodt, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

EP 1 308 713 A1

**ANNEX TO THE EUROPEAN SEARCH REPORT
ON EUROPEAN PATENT APPLICATION NO.**                    EP 01 20 4187

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2002

| Patent document cited in search report | | Publication date | Patent family member(s) | | Publication date |
|---|---|---|---|---|---|
| US 5844123 | A | 01-12-1998 | GB | 2289944 A | 06-12-1995 |
| | | | AT | 168470 T | 15-08-1998 |
| | | | AU | 697730 B2 | 15-10-1998 |
| | | | AU | 2621595 A | 21-12-1995 |
| | | | DE | 69503505 D1 | 20-08-1998 |
| | | | DE | 69503505 T2 | 22-04-1999 |
| | | | EP | 0763200 A1 | 19-03-1997 |
| | | | ES | 2122621 T3 | 16-12-1998 |
| | | | WO | 9533202 A1 | 07-12-1995 |
| | | | JP | 10505409 T | 26-05-1998 |
| | | | ZA | 9504388 A | 24-01-1996 |
| WO 9317324 | A | 02-09-1993 | AT | 186394 T | 15-11-1999 |
| | | | AU | 3570393 A | 13-09-1993 |
| | | | DE | 69326940 D1 | 09-12-1999 |
| | | | EP | 0627075 A1 | 07-12-1994 |
| | | | WO | 9317324 A1 | 02-09-1993 |
| | | | GB | 2278680 A ,B | 07-12-1994 |
| US 3942356 | A | 09-03-1976 | DE | 2518244 A1 | 01-04-1976 |
| | | | ES | 437180 A1 | 01-02-1977 |
| | | | FR | 2286378 A1 | 23-04-1976 |
| US 6138497 | A | 31-10-2000 | NONE | | |

EPO FORM P0459

For more details about this annex : see Official Journal of the European Patent Office, No. 12/82

10